# EUROPÄISCHE PATENTANMELDUNG

(11) **EP 0 541 999 A1**
(43) Veröffentlichungstag der Anmeldung: **19.05.1993**
(21) Anmeldenummer: 92117965.1
(22) Anmeldetag: 21.10.1992
(51) Int. Cl.: C07C 11/21, B01D 11/04

(54) **Verfahren und Vorrichtung zur Herstellung von Squalen aus Olivenölrückständen**

(30) Priorität: 15.11.1991 DE 4137733
(71) Anmelder: MÜLLER-EXTRACT-COMPANY GmbH, D-96450 Coburg (DE)
(72) Erfinder: Bondioli, Paolo, Dr., I-20133 Milano (IT); Lanzani, Armando, Dr., I-20133 Milano (IT); Fedeli, Enzo, Prof., I-20133 Milano (IT); Mossa, Andrea, Dr., I-20136 Milano (IT); Müller, Adam, Dr., Müller-Extract-Company Gmbh, W-8630 Coburg (DE)

(57) **Zusammenfassung**

Die Erfindung betrifft ein Verfahren und eine Vorrichtung zur Herstellung von Squalen aus Olivenöl-Rückständen, wobei vier Verfahrensstufen, die Verseifung, die Aufspaltung und Veresterung der Fettsäuren und die Extraktion durch überkritische Gase angewendet werden.
Zur Extaktion durch überkritische Gase gelangt ein zuvor mit nichtsäurehaltigem Katalysator verestertes Produkt, das in eine Hochdruck-Extraktionskolonne mit unabhängig voneinder temperierbaren Kolonnenzonen eingespritzt wird.

Mit diesem Verfahren und dieser Vorrichtung wird ein handelsfähiges Squalen mit einer Reinheit von mehr als 90 % gewonnen.

## Beschreibung

Die Erfindung betrifft ein Verfahren und eine Vorrichtung zur Herstellung von Squalen aus Olivenöl-Rückständen, wobei die Verfahrensstufen Verseifung, Aufspaltung, Veresterung der Fettsäuren und Extraktion durch überkritische Gase angewendet werden.

Es ist bekannt, daß die Herstellung von Squalen aus Fischleber durch Extraktion mit organischen Lösungsmitteln möglich ist.
Auf synthetischem Wege kann Squalen hergestellt werden aus hexaphenil 1,4 butandiyldiphosphonium-dibromid und 6,10 dimethil-5,9-undecadien-2-on.

Produkte von Fischleber in kommerziell verwertbaren Mengen sind nicht völlig geruchsfrei und enthalten noch eine Menge unangenehmer Geschmackskomponenten.
Squalen, gewonnen auf synthetischem Wege, ist sehr teuer, weil der geforderte Reinheitsgrad für eine kommerzielle Verwertung nicht zu erreichen ist.

Ferner ist durch das spanische Patent Nr. 555,112 bekannt, daß Squalen gewonnen werden kann durch Verseifung und Veresterung der Ölrückstände unter Benutzung eines säurehaltigen Katalysators während der Veresterung. Nach diesem Verfahren jedoch erhält man unerwünschte isomerisierte Squalenprodukte.

Gegenstand der vorliegenden Erfindung ist es, mit Hilfe eines besonderen Verfahrens und einer besonderen Vorrichtung eine höhere Ausbeute an gereinigtem Squalen zu erhalten, frei von fremdartigem Geruch oder Geschmack. Erfindungsgemäß wurde gefunden, daß ein hochwertiges Squalen in seiner natürlichen Zusammensetzung für kommerzielle Zwecke erhalten wird unter Anwendung von vier Verfahrensstufen:
1.
   Die Verseifung, die durch alkalisches Hydroxid als Startmaterial (Olivenöl-Rückstände) erreicht wird.
2.
   Die Fettsäuren-Aufspaltung: Zur alkalischen Hydroxid-Lösung von Stufe 1 werden soviel Mineralsäuren hinzugefügt, um die freien Fettsäuren von ihren Seifen abzutrennen.
3.
   Die Veresterung: Zu der organischen Schicht, die wie oben beschrieben erhalten wurde, wird Glyzerin und ein nichtsäurehaltiger Katalysator hinzugefügt.
4.
   Das veresterte Produkt wird nunmehr durch ein überkritisches Gas in eine eigens angefertige Vorrichtung extrahiert, wodurch ein hochwertiges Squalen gemäß der Erfindung erhalten wird.

Wenn die Veresterung mit Hilfe eines säurehaltigen Katalysators und anschließend die CO₂-Extraktion in allgemein bekannten Vorrichtungen durchgeführt wird, werden isomerisierte Squalen-Produkte von geringer Qualität erhalten.

Es ist überraschend festzustellen, daß ein hochwertiges Squalen mit einem Gehalt von mehr als 90 % in seiner natürlichen Zusammensetzung ohne Isomere erhalten wird, wenn die Veresterung mit einem nichtsäurehaltigen Katalysator z.B. Zinkpulver und unter Benutzung einer eigens konstruierten Extraktionskolonne für die Extraktion mit überkritischen Gasen durchgeführt wird.

Das veresterte Produkt gemäß der Erfindung wird eingespritzt in die Mitte einer Hochdruck-Extraktionskolonne, die eigens mit unterschiedlichen Segmenten und entsprechendem Doppelmantel ausgestattet ist (s.a. Zeichnung unter A, B, C, D, E, F, G).
Durch das überkritische Gas z.B. CO₂ wird Squalen gelöst, abgetrennt von den übrigen Bestandteilen und schließlich im Abscheidebehälter S gesammelt.
Die nicht gelösten Bestandteile des veresterten Produktes werden als Rückstand in der Sumpfzone G der Kolonne erhalten.

Die Einspritzung des veresterten Produktes kann sowohl am Kopfsegment als auch in einem anderen Segment der Kolonne erfolgen. Wichtig ist, daß das überkritische Gas wie z.B. CO₂ eingeführt wird an einem Punkt der Kolonne, sodaß die CO₂ im Gegenstrom von unten nach oben fließen kann.

Ferner ist die Art der Packungen in der Kolonne verantwortlich für einen optimalen Kontakt zwischen Produkt und überkritischem Gas. Nach dieser Verfahrensweise wird Squalen in CO₂ gelöst und über den Kopf der Kolonne bis zum Abscheider S befördert.
Das veresterte, vom Squalen abgetrennte Produkt wird als Rückstand in der Sumpfzone G der Kolonne gesammelt.

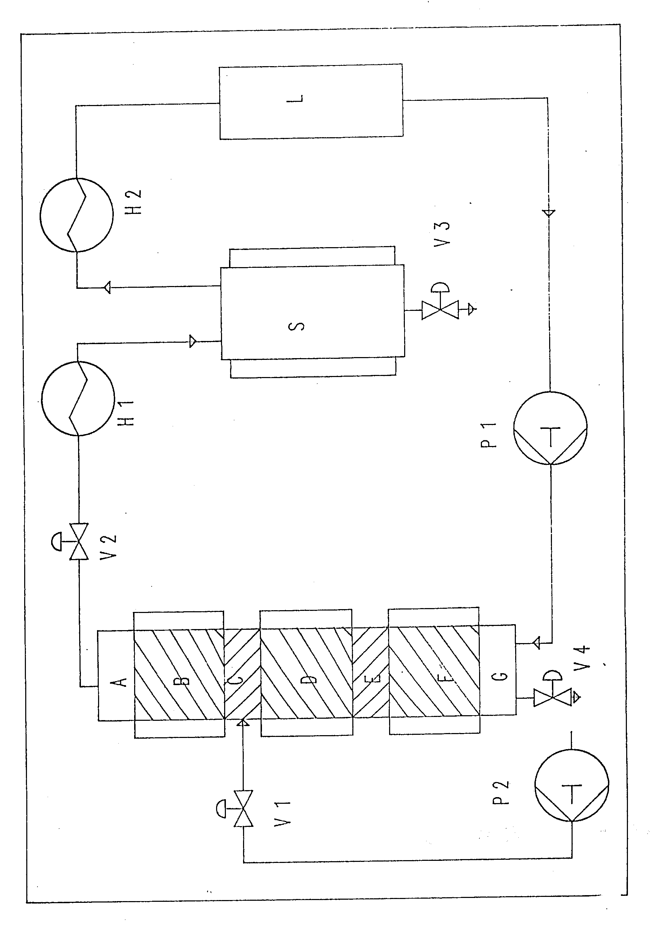

Zur Durchführung des Verfahrens gemäß der Erfindung ist es wichtig, daß die Kolonne mindestens zwei Segmente mit unterschiedlich voneinander arbeitenden Temperaturzonen besitzt. Bevorzugt werden drei oder mehr Segmente, die unabhängig voneinander temperierbar sind und mit unterschiedlichen Temperaturen arbeiten.
Im untersten Segment, das direkt über der Sumpfzone liegt, kann die Temperatur des Extraktionsgases unterkritisch, nahe dem kritischen Punkt, oder überkritisch sein. Der Extraktionsdruck ist in allen Fällen überkritisch.
Im nächstfolgenden Segment (z.B. D der Zeichnung), in welchem beispielsweise die Zuführung des veresterten Gemisches erfolgt, kann die Extraktionstemperatur ebenfalls unterkritisch, nahe dem kritischen Punkt, oder überkritisch sein. Der Extraktionsdruck ist auch hier immer überkritisch. In den weiter oben gelegenen Segmenten ( z.B. B der Zeichnung) müssen die Extraktionstemperatur und der Extraktionsdruck überkritisch sein, um evtl. noch nicht gelöste Squalenreste abzutrennen.

Die kritischen Daten für die benutzten überkritischen Gase sind bekannt, z.B. die kritische Temperatur für CO₂ ist +31,3^{o} C und 73,8 bar für den Druck.
Um das Verfahren gemäß der Erfindung ausführen zu können, wird die Vorrichtung gemäß der nachfolgenden Zeichnung benutzt. Hierbei ist zu bemerken, daß die Anzahl der Segmente mehr, aber auch weniger als drei sein kann.
Die Kolonne besteht aus den Segmenten B D F und den Zwischensegmenten A C E G, die durch Flansche miteinander verbunden sind. Die Kolonne ist gefüllt mit Packungen, z.B. Sulzer-Packungen. Die Segmente E B D F haben je einen Doppelmantel, der unabhängig und einzeln temperiert werden kann.

Die Pumpe P₁ saugt flüssige CO₂ vom Verflüssiger L und bringt diese auf den gewünschten überkritischen Druck. Die in bezug auf Druck überkritische CO₂ strömt vom Segment G zum Kopf der Kolonne A. Die in bezug auf Temperatur unterkritische oder überkritische CO₂ wird geregelt und gesteuert durch die den einzelnen Segmenten B D F eigenen Doppelmantelsysteme.
Das veresterte Produkt wird eingeführt am oberen Teil des Segmentes D oder des Zwischensegmentes C der Zeichnung unter Benutzung der Dosierpumpe P₂ und des Ventiles V₁. Das Startmaterial fließt kontinuierlich von P₂ zu C.

Die Erfindung ist nicht begrenzt auf diesen Punkt der Einführung des Startmaterials. Gemäß der Erfindung ist es möglich, einen anderen Einführungspunkt an der Kolonne zu wählen, entweder tiefer oder höher als Punkt C.

In den Segmenten C D E F ist die Abtrennung des Squalen noch nicht vollständig von den Begleitsubstanzen.

Gemäß der Erfindung findet die restliche Lösung der noch nicht in CO₂ gelösten Squalenanteile im Segment B statt. Mit den beschriebenen Verfahrensbedingungen wird ein in CO₂ gelöstes Kopfprodukt erhalten, das vom Kopf der Kolonne A zu dem proportional gesteuerten Ventil V₂ strömt, wo der Extaktionsdruck reduziert wird.

In dem sich anschließenden Wärmetauscher H₁ wird ebenfalls die Extraktionstemperatur reduziert. Unter diesen Bedingungen gelangt der Extrakt in den Separator S, wo die Trennung von der CO₂ erfolgt.
Der gewonnene Extrakt enthält nahezu das gesamte Squalen und kann kontinuierlich durch das Ventil V₃ aus dem Abscheidebehälter abgeführt werden. Das Sumpfprodukt, nahezu frei von Squalen, wird im Segment G gesammelt und kann kontinuierlich durch Ventil 4 ausgetragen werden.
Die CO₂ im Abscheider S ist frei von Extrakt, wird im Wärmetauscher H₂ verflüssigt und fließt in den sogen. Verflüssiger L, der im wesentlichen einen Sammelbehälter für verflüssigte CO₂ darstellt.

Das Kohlendioxid kann wieder durch die Pumpe P₁ für einen neuen Extraktionszyklus eingesetzt werden.

### Beispiel

500 g feste Rückstände aus der Olivenölherstellung werden unter Rückfluß mit 500 ml Methanol, 500 ml Wasser und mit einem Überschuß an NAOH oder KOH, berechnet auf der Basis der Verseifungszahl behandelt. Die Behandlungsdauer beträgt etwa drei Stunden. Anschließend wird die alkalische Lösung mit 85 % Phosphorsäure versetzt. Die Fettsäurenspaltung wird bei einer Reaktionstemperatur von 70^{o} C durchgeführt. Die Vollständigkeit der Fettsäurenspaltung ist erreicht, wenn zwei nicht mischbare Flüssigkeitsschichten zu beobachten sind.

Die organische Schicht des behandelten Produktes - wie oben beschrieben - wird übergeführt in einen 1.000 ml Glaszylinder, der mit Magnetrührer und Vakuumpumpe verbunden ist.

Nach der Wasserentfernung bei 100^{o} C unter Vakuum (20 mm H_{g}) wird die Temperatur bis auf 180^{o} C gesteigert und Glyzerin und Zinkpulver 1 % als Katalysator hinzugefügt. Der Gehalt an Glyzerin ist berechnet auf Basis des freien Fettsäurenanteiles.
Während der Veresterung muß das Vakuum aufrecht erhalten bleiben.
Die Behandlung ist beendet, wenn der freie Fettsäurenanteil weniger als 1 % ist.

300 ml des vorliegenden veresterten Produktes mit einem Gehalt von 28 % Squalen als Startmaterial werden in die beschriebene Produktionskolonne injiziert, die mit CO₂ als überkritisches Gas arbeitet.
Der Extraktionsdruck ist 150 bar, die Extraktionstemperatur im mittleren Segment (D) 40^{o} C, im oberen Segment (B) 50^{o} C, im tiefer gelegenen Segment (F) 30^{o} C.

Die Ergebnisse sind:

| | |
|---|---|
| Extraktgehalt | 28,3 % |
| Squalen im Extrakt | 90,0 % |
| Squalen im Rückstand | 2,9 % |
| Mono- und Diglyceride im Extrakt | 7,0 % |
| Extraktausbeute | 93,9 % |

Die Durchflußrate an überkritischem CO₂ beträgt 100 kg/h.

Der Umfang der vorliegenden Erfindung ist nicht begrenzt auf die Bedingungen des Beispieles.
Durch Änderung der o.gen. Bedingungen, insbesondere der Verfahrensbedingungen im Hinblick auf Extraktionsdruck und / oder Temperatur und / oder Durchflußmenge des überkritischen Gases ist es möglich, den Prozeß und die erhaltenen Daten zu optimieren.

## Patentansprüche

1. Verfahren zur Herstellung von Squalen aus Resten der Olivenöl-Herstellung durch Verseifen, Spaltung der Fettsäuren, Veresterung und Abtrennung durch ein überkritisches Gas, dadurch gekennzeichnet,
daß die Veresterung unter Benutzung eines nicht säurehaltigen Katalysators und die Abtrennung durch ein überkritisches Gas unter Verwendung einer Hochdruck-Extraktionskolonne durchgeführt wird.

2. Verfahren nach Anspruch 1 dadurch gekennzeichnet,
daß der Extraktionsdruck in der Kolonne 75 - 350 vorzugsweise 110 - 160 bar beträgt.

3. Verfahren nach Anspruch 2 dadurch gekennzeichnet,
daß in der Kolonne gleichzeitig für CO₂ überkritische wie unterkritische Temperaturen vorzugsweise von +20^{o}C bis +80^{o}C.

4. Vorrichtung zur Durchführung des Verfahrens nach den Ansprüchen 1 - 3 dadurch gekennzeichnet, daß die Hochdruck Extraktionskolonne aus mindestens 2, zweckmäßigerweise aus 3 oder mehreren mit Doppelmantel ausgestatteten Segmenten besteht.

5. Vorrichtung zur Durchführung des Verfahrens nach den Ansprüchen 1 - 4 dadurch gekennzeichnet,
daß die einzelnen Segmente der Hochdruck-Extraktionskolonne unabhängig voneinander temperierbar sind.
